(19) Europäisches Patentamt
European Patent Office
Office européen des brevets

(11) **EP 4 321 206 A2**

(12) **EUROPEAN PATENT APPLICATION**

(43) Date of publication:
**14.02.2024 Bulletin 2024/07**

(21) Application number: **23214277.8**

(22) Date of filing: **02.09.2021**

(51) International Patent Classification (IPC):
**A61N 1/36** $^{(2006.01)}$

(52) Cooperative Patent Classification (CPC):
**A61N 1/375; A61N 1/36038; A61N 1/3718**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2020 EP 20194214**

(62) Document number(s) of the earlier application(s) in accordance with Art. 76 EPC:
**21194598.5 / 3 964 259**

(71) Applicant: **MED-EL Elektromedizinische Geräte GmbH**
**6020 Innsbruck (AT)**

(72) Inventor: **ZIMMERLING, Martin**
**6082 Patsch (AT)**

(74) Representative: **Lucke, Andreas**
**Boehmert & Boehmert**
**Anwaltspartnerschaft mbB**
**Pettenkoferstrasse 22**
**80336 München (DE)**

Remarks:
This application was filed on 05-12-2023 as a divisional application to the application mentioned under INID code 62.

(54) **HOLDING MAGNETS AND MAGNET SYSTEM FOR IMPLANTABLE SYSTEMS OPTIMIZED FOR MRI**

(57) The present invention relates to at least partially implantable devices such as partly implantable hearing devices, for example cochlear implants, and specifically, to implantable magnets for interaction with external magnets in such devices. Embodiments of the present invention are directed to a magnet arrangement for a hearing implant device. An implant device contains signal processing circuitry configured for receiving an implant communications signal transmitted from an external transmitting coil through overlying skin of an implanted patient, and the implant device includes an outermost surface adapted to lie between the overlying skin and underlying skull bone of the implanted patient. There is a magnet case within the implant device, and the magnet case is configured to be rotatable about a case rotation axis which is at least approximately perpendicular to said outermost surface of the implant device. An implant magnet arrangement is located within the magnet case and configured to cooperate with a corresponding external holding magnet in an external device located over the overlying skin to magnetically hold the external device against the overlying skin.

Fig. 14

EP 4 321 206 A2

**Description**

FIELD OF THE INVENTION

The present invention relates to at least partially implantable devices such as partly implantable hearing devices, for example cochlear implants, and specifically, to implantable magnets for interaction with external magnets in such devices.

BACKGROUND ART

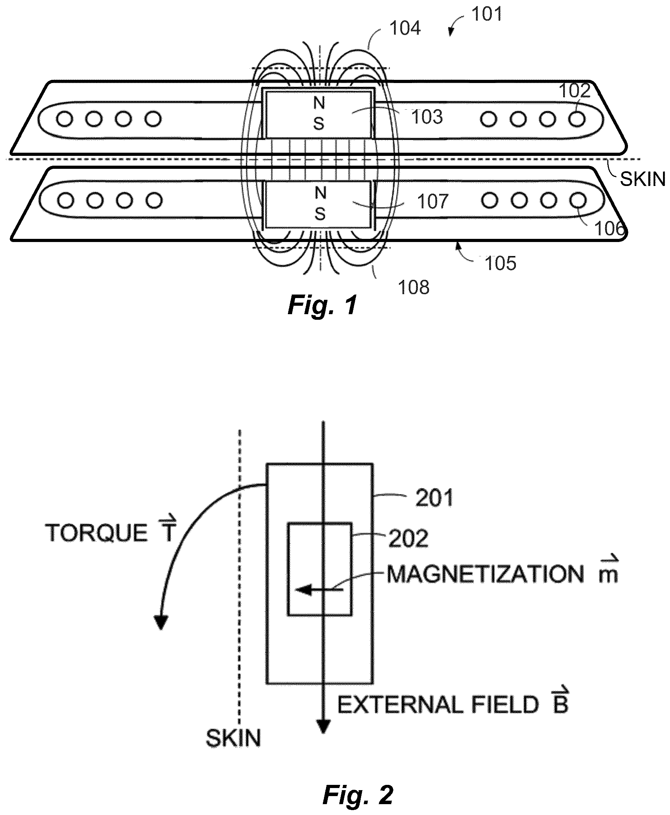

Some hearing implants such as Middle Ear Implants (MEI's) and Cochlear Implants (CI's) employ cooperating attachment magnets located in the implant and the external part to magnetically hold the external part in place over the implant. For example, as shown in Fig. 1, a typical cochlear implant system may include an external transmitter device **101** containing transmitting coil **102** and an external attachment magnet **103**. The external attachment magnet **103** has a conventional cylindrical disc-shape and a north-south magnetic dipole having an axis that is perpendicular to the skin of the patient to produce external magnetic field lines **104** as shown. Implanted under the patient's skin is a corresponding receiver assembly **105** having similar receiving coil **106** and an implant magnet **107**. The implant magnet **107** also has a cylindrical disc-shape and a north-south magnetic dipole having a magnetic axis that is perpendicular to the skin of the patient to produce internal magnetic field lines **108** as shown. The internal receiver device **105** is surgically implanted and fixed in place within the patient's body. The external transmitter device **101** is placed in proper position over the skin covering the internal receiver assembly **105** and held in place by interaction between the internal magnetic field lines **108** and the external magnetic field lines **104.** Rf signals from the transmitter coil **102** couple data and/or power to the receiving coil **106** which is in communication with an implanted processor module (not shown).

One problem arises when the patient undergoes Magnetic Resonance Imaging (MRI) examination. Interactions occur between the implant magnet and the applied external magnetic field for the MRI. As shown in Fig. 2, the direction of the magnetization $\vec{\mathbf{m}}$ of the implant magnet **202** is essentially perpendicular to the skin of the patient. In this example, the strong static magnetic field $\vec{\mathbf{B}}$ from the MRI creates a torque $\vec{\mathbf{T}}$ on the internal magnet **202,** which may displace the internal magnet **202** or the whole implant device **201** out of proper position. Among other things, this may damage the adjacent tissue in the patient. In addition, the external magnetic field $\vec{\mathbf{B}}$ from the MRI may reduce or remove the magnetization $\vec{\mathbf{m}}$ of the implant magnet **202** so that it may no longer be strong enough to hold the external transmitter device in proper position. The implant magnet **202** also may cause imaging artifacts in the MRI image, there may be induced voltages in the receiving coil, and hearing artifacts due to the interaction of the external magnetic field $\vec{\mathbf{B}}$ of the MRI with the implanted device. Torque and forces acting on the implant magnet and demagnetization of the implant magnet are especially an issue with MRI field strengths at 1.5 Tesla and higher.

Thus, for many existing implant systems with magnet arrangements, it is common to either not permit MRI, or limit use of MRI to lower field strengths. Other existing solutions include use of a surgically removable magnets, spherical implant magnets (e.g. U.S. Patent 7,566,296, incorporated herein by reference in its entirety), and various ring magnet designs (e.g., U.S. Patent Publication 2011/0022120 incorporated herein by reference in its entirety).

U.S. Patent 8,634,909 (incorporated herein by reference in its entirety) discloses an implant magnet having a magnetic dipole moment direction that is parallel to the end surfaces of a disc shaped implant magnet-that is, perpendicular to the conventional magnetic dipole moment direction of a disc-shaped implant magnet. The magnet is then held in a magnet receptacle that allows the magnet to rotate about its center axis in response to an external magnetic field such as from an MRI to realign and minimize creating torque. But this rotation is only possible around a single axis.

It also has been suggested to use a set of multiple cylindrical magnets which are magnetized perpendicular to the cylinder axis and rotatable about their cylinder axis embedded into a magnet frame and case which can rotate around the central axis of the case (see e.g., WO2O17/105510, which is incorporated herein by reference in its entirety). In that approach two or more diametrically-magnetized cylindrical magnets always align in a configuration where one north pole is oriented next to an adjacent south pole and vice versa. One disadvantage of this configuration is, that the cylindrical magnets together behave like a single disc shaped magnet as disclosed in the '909 patent with magnetization direction parallel to the skin surface - unless a very strong external magnetic field is applied -, but due to a relatively poor filling factor have only a small combined magnet volume, thus requiring an extra-large or extra-strong external magnet. On the other hand, using two or more cylindrical magnets does allow a relatively slim implant magnet design.

## SUMMARY OF THE INVENTION

Embodiments of the present invention are directed to a magnet arrangement for a hearing implant device. An implant device contains signal processing circuitry configured for receiving an implant communications signal transmitted from an external transmitting coil through overlying skin of an implanted patient, and the implant device includes an outermost surface adapted to lie between the overlying skin and underlying skull bone of the implanted patient. There is a magnet case within the implant device, and the magnet case is configured to be rotatable about a case rotation axis which is at least approximately perpendicular to said outermost surface of the implant device. An implant magnet arrangement is located within the magnet case and configured to cooperate with a corresponding external holding magnet in an external device located over the overlying skin to magnetically hold the external device against the overlying skin. The implant magnet arrangement includes multiple cylindrical magnets, each with a center cylinder axis that is perpendicular to the case axis, and each cylindrical magnet is configured to be rotatable about its center cylinder axis. Each cylindrical magnet has an outer cylindrical surface with a north magnetic pole and a south magnetic pole, and a north magnetic direction is defined by a radial vector extending from the center cylinder axis to the north magnetic pole, and a south magnetic direction is defined by a radial vector extending from the south magnetic pole to the center cylinder axis. The north magnetic pole and the south magnetic pole are arranged with respect to each other so as not to lie on a common diameter through the center cylinder axis such that the north magnetic direction and the south magnetic direction form a magnetic angle less than 180 degrees with a vertex at the center cylinder axis.

In further specific embodiments, the implant magnet arrangement may be configured for the cylindrical magnets to magnetically align with respect to each other to create a common line of magnetic flux through the cylindrical magnets, the magnet case, and the overlying skin to cooperate with the external holding magnet. There also may be one or more diametrically magnetized supplemental cylindrical magnets located between the cylindrical magnets and configured to couple the common line of magnetic flux between the cylindrical magnets. In addition or alternatively, there may be soft magnetic material located between the cylindrical magnets and configured to couple the common line of magnetic flux between the plurality of cylindrical magnets.

The implant magnet arrangement may be configured to respond to a strong external magnetic field by rotation of the magnet case about the case rotation axis and rotation of the cylindrical magnets about their respective center cylinder axes so as to minimize net torque imparted to the implant device. The magnetic angle may be between 90 degrees and 140 degrees. Each cylindrical magnet may be configured to be fully rotatable about the center cylinder axis through a complete rotation range of 360 degrees. Or each cylindrical magnet may be configured to be limitedly rotatable about the center cylinder axis through a limited rotation range of less than 180 degrees. For example, the limited rotation range may be 90 degrees.

A second aspect of the invention relates to an implant device containing signal processing circuitry configured for receiving an implant communications signal transmitted (e.g. from an external transmitting coil) through overlying skin of an implanted patient, wherein the implant device includes an outermost surface adapted to lie between the overlying skin and underlying skull bone and at least approximately parallel to the skin of the implanted patient; and an implant magnet configured to cooperate with an external holding magnet in an external device to be located over the overlying skin to magnetically hold the external device against the overlying skin. According to the second aspect of the invention, said implant magnet has a north magnetic pole, a south magnetic pole, and as a whole has an overall magnetic dipole moment that is parallel to or at an angle of 30° or less, preferably 20° or less with respect to said outermost surface. Moreover, said implant magnet has a north end portion including said north magnetic pole and a south end portion including said south magnetic pole, said north and south end portions each being formed from permanent magnetic material and each having an individual magnetic dipole moment that is inclined with respect to said overall magnetic dipole moment, wherein said individual magnetic dipole moment in said north end portion is inclined with respect to said overall magnetic dipole moment such as to have a component pointing towards said outermost surface, and said individual magnetic dipole moment in said south end portion is inclined with respect to said overall magnetic dipole moment such as to have a component pointing away from said outermost surface. The north and south end portions of the implant magnet may be fixedly attached directly with each other, or may each be fixedly attached to an intermediate portion of said implant magnet.

According to the second aspect of the invention, the implant magnet "as a whole" has an "overall magnetic dipole moment" which is at least approximately parallel to the outermost surface, or in other words, approximately parallel to the skin in the implanted state. As the skilled person will appreciate, the magnetic dipole moment $m$ of a magnetic body as a whole is a macroscopic quantity defining the "strength" of the magnetic dipole. When arranged in an external magnetic field having a flux density $B$, a torque $\tau$ is generated that corresponds to the vector product of the magnetic dipole moment $m$ and the flux density $B$, i.e. $\tau = m \times B$. Accordingly, the overall magnetic dipole moment $m$ of the implant magnet can be readily determined when placed in an external magnetic field: the implant magnet will orient itself to bring the dipole moment $m$ in alignment with the flux density $B$ of the external magnetic field, thereby revealing the direction of the magnetic dipole moment $m$, while its magnitude is defined by the size of the torque needed to rotate the implant magnet out of this alignment. The magnetic dipole moment of the implant magnet as a whole determines how the internal magnet reacts to the external magnetic field in an MRI device.

However, according to the invention, the implant magnet has north and a south end portions including the north and south magnetic poles, respectively, each having an individual magnetic dipole moment that is inclined with respect to said overall magnetic dipole moment. In these north and south end portions, the respective magnetizations are hence not aligned with the overall magnetic dipole moment of the implant magnet as a whole. The magnetization of a material is designated as a vector field **M** that expresses the density of magnetic dipole moments in the magnetic material, i.e.

$$M = \frac{dm}{dV}$$

, where *dm* is the elementary magnetic moment and *dV* is the corresponding volume element. In other words, the magnetic moment *m* associated with a magnet is the space integral of the magnetization **M** over the magnet's volume, i.e. $m = \iiint M \, dV$. As used herein, a "vector" is simply understood as a physical object that has a magnitude and a direction. No distinction between vectors and pseudovectors according to their transformation properties is made herein. Vectors are generally represented by symbols in bold font.

In the invention, the north and south end portions are each formed from permanent magnetic material. A "permanent magnetic material" as understood herein has a broad meaning, but is in any case distinguished from magnetic soft materials such as soft iron as it is used in the art for pole shoes or the like. In particular, the permanent magnetic material in the north and south end portions should have an intrinsic magnetic coercivity $H_{Ci}$ of more than 200 A/m, preferably of more than 500 A/m, more preferably more than 800 A/m and most preferably more than 1000 A/m. The individual magnetic dipole moment in said north end portion has a component pointing towards said outermost surface, and the individual magnetic dipole moment in said south end portion has a component pointing away from said outermost surface. The inventor has found that this way, the magnetic attraction or holding force applied to an external magnet of an external device can be significantly increased as compared to a prior art implant magnet of same size and material which is homogeneously magnetized in a direction parallel to the skin throughout its volume, while still allowing for an overall magnetic moment that is parallel to the skin.

In preferred embodiments, the implant magnet is rotatable around a rotation axis that is perpendicular to said outermost surface, or deviates from perpendicular by less than 30°, preferably less than 20°, wherein in each available rotational position of said implant magnet upon rotation around its rotation axis, said overall magnetic dipole moment is parallel to or at an angle of 30° or less, preferably 20° or less with respect to said outermost surface.

Preferably, said implant magnet has a shape that is rotationally symmetric around said rotation axis. In a preferred embodiment, said implant magnet has an outer end surface facing said outermost surface of said implant device and an inner end surface facing away from said outermost surface, wherein one or both of said inner and outer end surfaces are perpendicular to said rotation axis. For example, the implant magnet may have a disc shape.

In preferred embodiments, said implant magnet has a planar outer end surface, to optimally make use of the limited space in the implant device.

In a preferred embodiment, the angle of inclination between each individual magnetic dipole moment in said north and south end portions with respect to the overall magnetic dipole moment is ≤ 50°. This will allow for safely avoiding a situation in which the implant magnet could be inadvertently weakened or de-magnetized in a strong external MRI field in case the patient does not hold his or her head straight during the MRI procedure, but for example tilted to one side by e.g. up to 30°.

In a preferred embodiment, said implant magnet has an average diameter $d_I$ in a direction parallel to the overall magnetic dipole moment and an average thickness $h_I$ in a direction perpendicular to said outermost surface, wherein in one or both of said north and south end portions, said individual magnetic dipole moment is inclined with respect to said overall magnetic dipole moment by an angle a, wherein

$$arctan\,(h_I \,/\, (d_I \,/2)) \; -15° \leq \alpha \leq arctan\,(h_I \,/\, (d_I \,/2)) \; +7°,$$

preferably

$$arctan\,(h_I \,/\, (d_I \,/2)) \; -10° \leq \alpha \leq arctan\,(h_I \,/\, (d_I \,/2)) \; +5°.$$

Herein, the angle $\alpha$ is measured in a plane that is perpendicular to the outermost surface. This angular range has been found to allow for a particularly good increase in the attachment force. For larger angles a, the distance between the north and south poles would decrease, which in turn would lead to an excessive decrease in holding force with distance from the implant magnet.

In some embodiments, said north and south end portions are directly adjacent with each other, and in particular each form one of two halves of said implant magnet. This embodiment leads to very good holding forces, and at the same time allows for comparatively easy manufacture.

In alternative embodiments, however, said north and south end portions of said implant magnet may be separated from each other by an intermediate portion having an individual magnetic dipole moment that is parallel to said overall magnetic dipole moment, or deviates from parallel by less than 10°, preferably less than 5°. This embodiment allows for comparatively large inclinations of the magnetization in the north and south end portions while at the same time avoiding magnetic short-circuits at the outer surface, thereby leading to very good holding forces.

In addition or alternatively, one or both of said north and south end portions of said implant magnet may have an outer section closer to said outermost surface and an inner section further away from said outermost surface, wherein an angle of inclination of the individual magnetic dipole moment with respect to the overall magnetic dipole moment in said outer section is less than in said inner section. This embodiment allows for an improved magnetic flux within the implant magnet while avoiding the distance of the north and south poles to decrease.

In a preferred embodiment, said implant magnet has an outer end surface facing said outermost surface of said implant device and an inner end surface facing away from said outermost surface. Moreover, a middle plane is defined to be located at equal distance from said outer and inner end surfaces, and said implant magnet preferably fulfils one or both of the following criteria (i) and (ii):

(i) at least 55 %, preferably at least 65 % of the total magnetic flux of a magnetic field generated outside of the implant magnet when placed in isolation in air or a vacuum is located on a side of said middle plane at which said outermost surface is located in the assembled state,
(ii) more than 50%, preferably more than 55% of the mass of the magnet is located on a side of said middle plane at which said outermost surface is located, wherein in particular, the edges of the magnet at the inner end surface are chamfered.

Criterion (i) defines the distribution of the magnetic flux generated by the implant magnet itself, i.e. when placed in isolation in air or vacuum. According to this criterion, the larger part of the magnetic flux is on one side of the middle plane, and this side is the side on which in the "assembled state", i.e. when the implant magnet is arranged in the implant device, the outermost surface of the implant device would be located. In other words, the implant magnet is devised so that by itself it already generates the bigger part of its flux in the region where it is needed for establishing an attractive holding force with an external device, i.e. more to the outside than to the inside of the body.

The second criterion (ii) specifies that more than half of the mass of the implant magnet is located on the side of the middle plane at which the outermost surface located. This again helps with generating the magnetic flux closer to the outside region than the inside region with respect to the implant magnet. One way of reducing the mass of the implant magnet towards the inside of the middle plane is by providing for chamfered edges at the inner end surface of the implant magnet. This shape also allows for a more favorable magnetic flux.

In a preferred embodiment, said north and south end portions are formed from anisotropic magnet elements each having a preferred magnetization direction, wherein said anisotropic magnet elements are joined with each other or with an intermediate portion arranged in between. Said preferred magnetization directions are arranged at an angle with respect to the overall dipole moment of the implant magnet as a whole. The anisotropic magnet elements can e.g. be made by applying an external magnetic field while the magnet is formed, which may for example involve sintering. The anisotropic magnets have the advantage that they allow for higher magnetizations in their final state. The anisotropic magnets will not have their final magnetic strength after manufacture yet, but will only acquire it after a final magnetization using a strong magnetic pulse. While an isotropic magnet can be magnetized by a strong magnetic pulse in any direction, the anisotropic magnets can only be magnetized in their preferred magnetization directions established upon manufacture. This is actually an advantage in the manufacturing process of the magnet as a whole, because the magnet can be assembled from anisotropic magnet elements with the preferred magnetization directions in the north and south end portions inclined with respect to the overall magnetization direction, but before the anisotropic magnet elements are fully magnetized. This makes the handling of the anisotropic magnet elements prior to the assembly, as well as the joining of the magnet elements much easier. It is then possible to fully magnetize the anisotropic magnet elements in the joint state by applying an external magnetic pulse aligned with the direction of the eventual overall magnetic dipole moment. During this magnetization process, the magnetization directions of the anisotropic magnet elements as part of the entire implant magnet will be preserved, while their strength will be increased.

In preferred embodiments, said implant magnet may have on at least part of the inner end surface (913) a layer of magnetic soft material, such as for example soft iron, applied. This may help to shield a magnetic field generated by the implant magnet itself toward the inside of the body and thereby may reduce artefacts during MRI-scanning.

In preferred embodiments, said implant magnet is a rare earth magnet, in particular a rare earth magnet comprising neodymium, samarium, terbium, dysprosium or holmium.

A further embodiment of the invention relates to an implant system comprising an implant device or magnet arrangement according to one of the preceding embodiments and an external device containing signal processing circuitry configured for transmitting an implant communications signal to said implant device, said external device comprising an innermost surface adapted to lie adjacent to said skin; and an external magnet or magnet assembly in said external device to be located over the overlying skin and magnetically configured to cooperate with an implant magnet or magnet arrangement as defined in any of the preceding embodiments such as to hold the external device against the skin.

The external magnet may be similar to the implant magnet, but may also be of a different design. In particular, since the external device can be taken off prior to an MRI procedure, the external magnet does not have to be specifically designed to cope with strong external MRI magnetic fields. Accordingly, the external magnet may be an arrangement of two magnets having magnetic dipole moments arranged perpendicular to the innermost surface of the external device, and hence to the skin, with their north and south poles arranged adjacent to the south and north poles of the implant magnet, respectively.

However, in some embodiments of the implant system, said external magnet or magnet arrangement may have a north magnetic pole, a south magnetic pole, and may further as a whole have an overall magnetic dipole moment that is parallel to or at an angle of 30° or less with respect to said innermost surface of said external device.

Moreover, the external magnet may be of a similar design as disclosed in one of the embodiments with respect to the internal magnet above. In particular, the external magnet may have a north end portion including said north magnetic pole and a south end portion including said south magnetic pole, said north and south end portions each being formed from permanent magnetic material and each having an individual magnetic dipole moment that is inclined with respect to said overall magnetic dipole moment of said external magnet, wherein said individual magnetic dipole moment in said north end portion has a component pointing towards said innermost surface of said external device, and said individual magnetic dipole moment in said south end portion has a component pointing away from said innermost surface of said external device.

In a preferred embodiment of said implant system, said external magnet is rotatable around a rotation axis that is perpendicular to said innermost surface of said external device or deviates from perpendicular by less than 30°, wherein in each available rotational position of said external magnet upon rotation around its rotation axis, said overall magnetic dipole moment is parallel to or at an angle of 30° or less with respect to said innermost surface, wherein said external magnet preferably has a shape that is rotationally symmetric around its rotation axis.

In preferred embodiments, said external magnet has a planar inner end surface facing said innermost surface of said external device.

In a preferred embodiment of the implant system, said external magnet has an average diameter $d_E$ in a direction parallel to the overall magnetic dipole moment and an average thickness $h_E$ in a direction perpendicular to said innermost surface of the external device, wherein in one or both of said north and south end portions, said individual magnetic dipole moment is inclined with respect to said overall magnetic dipole moment by an angle a, wherein

$$arctan\,(h_E\,/\,(d_E\,/2))\,\text{-}15^o \leq \alpha \leq arctan\,(h_E\,/\,(d_E\,/2))\,+\,7^o,$$

preferably

$$arctan\,(h_E\,/\,(d_I\,/2))\,\text{-}10^o \leq \alpha \leq arctan\,(h_E\,/\,(d_E\,/2))\,+\,5^o.$$

The advantages of these angular ranges are similar to those explained above with reference to the implant magnet. Similar to the case of the implant magnet, the angle $\alpha$ is measured in a plane that is perpendicular to the innermost surface of the external device, or in other words, in a plane that is at least approximately perpendicular to the skin.

In some embodiments, said north and south end portions of said external magnet are directly adjacent with each other, and in particular each form one of two halves of said external magnet. In alternative embodiments, said north and south end portions of said external magnet are separated from each other by an intermediate portion having an individual magnetic dipole moment that is parallel to said overall magnetic dipole moment of said external magnet or deviates from parallel by less than 10°, preferably less than 5°.

In a preferred embodiment of the implant system, one or both of said north and south end portions of said implant magnet have an inner section closer to said innermost surface of said external device and an outer section further away from said innermost surface of said external device, wherein an angle of inclination of the individual magnetic dipole moment with respect to the overall magnetic dipole moment in said inner section is less than in said outer section.

In a preferred embodiment, said external magnet has an inner end surface facing said innermost surface of said external device and an outer end surface facing away from said innermost surface of said external device, wherein

EP 4 321 206 A2

a middle plane is defined to be located at equal distance from said outer and inner end surfaces of said external device, and wherein said external magnet fulfils one or both of the following criteria (i) and (ii):

> (i) at least 55 %, preferably at least 65 % of the total magnetic flux of a magnetic field generated outside of the external magnet when placed in isolation in air or a vacuum is located on a side of said middle plane at which said innermost surface of the external device is located in the assembled state,
> (ii) more than 50%, preferably more than 55% of the mass of the external magnet is located on a side of said middle plane at which said innermost surface is located, wherein in particular, the edges of the magnet at the inner end surface are chamfered.

In a preferred embodiment, said north and south end portions of said external magnet are formed from anisotropic magnet elements each having a preferred magnetization direction, wherein said anisotropic magnet elements are joined with each other or with an intermediate portion arranged in between, wherein said preferred magnetization directions are arranged at an angle with respect to the overall dipole moment of the external magnet as a whole.

In a preferred embodiment, said external magnet is a rare earth magnet, in particular a rare earth magnet comprising neodymium, such as neodymium-ion-boron magnets, or comprising samarium, such as samarium-cobalt magnets, or comprising terbium, or dysprosium or holmium or combinations thereof.

Embodiments of the present invention also include a hearing implant system containing a magnet arrangement according to any of the foregoing.

BRIEF DESCRIPTION OF THE DRAWINGS

Figure 1 shows portions of a typical cochlear implant system and the magnetic interaction between the implant magnet and the external implant magnet.

Figure 2 illustrates the force interactions that can occur between an implant magnet and the applied external magnetic field for an MRI system.

Figure 3A shows an example of a cylindrical implant magnet with a V-shaped magnetic angle according to an embodiment of the present invention.

Figure 3B shows an example of a cochlear implant device with two implant magnets of the type shown in Fig. 3A.

Figures 4A and 4B show how the magnetic fields of the implant magnets align to cooperate with an external holding magnet.

Figures 5A and 5B show how the magnetic fields of the implant magnets align in the presence of an MRI magnetic field.

Figures 6A and 6B show an example of an embodiment with a diametrically magnetized supplemental cylindrical magnet located between the cylindrical magnets.

Figures 7A and 7B show an example of an embodiment with soft magnetic material located between the cylindrical magnets.

Figure 8 shows an embodiment of cylindrical implant magnets that are configured to be limitedly rotatable through a limited rotation range according to an embodiment of the present invention.

Figures 9A-9C show orientations of the cylindrical implant magnets for different orientations of an external MRI magnetic field.

Figures 10A-10C show orientations of the cylindrical implant magnets for reverse magnetization in a reduced rotatability embodiment of the present invention.

Figure 11 shows a further example of a cochlear implant device using an implant magnet with north and south end portions having individual magnetic dipole moments that are inclined with respect to the overall magnetic at the moment of the implant magnet.

Figure 12 is a perspective view of an implant magnet of the type used in the device of Figure 11.

Figure 13 is a side view of the device of Figure 11.

Figure 14 is a schematic sectional view showing an implant device and an external device with corresponding magnets.

Figure 15 is a schematic sectional view showing magnets of an implant and an external device.

Figure 16 illustrates the direction of the magnetic flux generated by an ordinary implant magnet that is homogeneously magnetized.

Figure 17 illustrates the direction of the magnetic flux generated by an implant magnet of the invention having north and south end portions having individual magnetic dipole moments that are inclined with respect to the overall magnetic dipole moment of the implant magnet.

Figure 18 illustrates the flux density of the ordinary implant magnet of Figure 16.

Figure 19 illustrates the flux density of the implant magnet of the invention according to Figure 17.

Figure 20 shows the magnetic flux density generated upon the interaction of a homogeneously magnetized external magnet and the ordinary implant magnet of Figure 16.

Figure 21 shows the magnetic flux density generated upon the interaction of homogeneously magnetized external magnet and the implant magnet of the invention according to Figure 17.

Figure 22 is a schematic sectional view of an implant magnet and an external magnet according to a further embodiment of the invention.

Figure 23 is a schematic view illustrating the torque applied to a conventional implant magnet and an implant magnet of the invention by an external magnetic field.

DETAILED DESCRIPTION OF SPECIFIC EMBODIMENTS

A larger distance between the two magnetic poles has the advantage that the attractive magnetic force for an external magnet does not decrease so steeply with increasing distance between the magnets. Embodiments of the present invention are directed to an improved implant magnet arrangement that uses two cylindrical implant magnets with magnetization direction having a V-shaped magnetic angle. These magnets are mounted in the implant device such that a "strong" side (i.e. the side with high magnetic flux) faces at least partly towards the overlying skin. Both magnets are mounted inside a magnet case in which they can turn also around the rotation axis of the case.

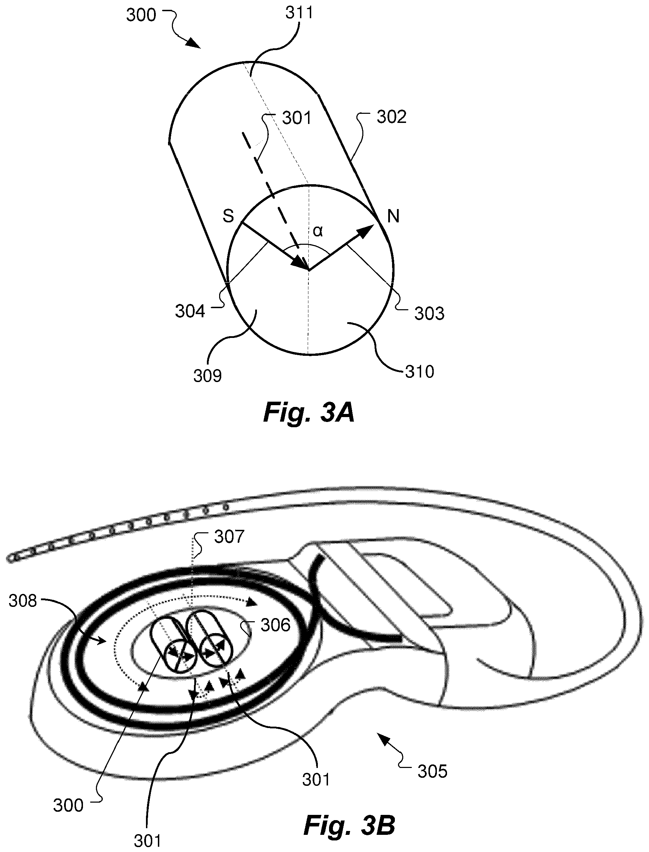

Figure 3A shows an example of a cylindrical implant magnet **300** with a V-shaped magnetic angle α according to an embodiment of the present invention, and Figure 3B shows an example of a cochlear implant device **305** with two implant magnets **300** of the type shown in Fig. 3A. The cochlear implant device **305** contains signal processing circuitry (not shown) configured for receiving an implant communications signal transmitted from an external transmitting coil through overlying skin of an implanted patient, and an outermost surface **308** adapted to lie between the overlying skin and underlying skull bone of the implanted patient.
There is a magnet case **306** within the implant device **305** with a case rotation axis **307** that is perpendicular to the outermost surface **308** of the implant device **305.** The magnet case **306** is configured to be rotatable about the case rotation axis **307.** Typically the magnet case **306** is surrounded by a receiver coil of the implant device **305.** The magnet case **306** may be metallic (e.g. made of titanium), or it may be made of a biocompatible non-metallic material (e.g. PEEK, FEP, PTFE, PSU, etc.) and may be coated (e.g. with Parylene). The magnet case **306** may be adapted to facilitate long-term hermetic encapsulation, and/or it may be adapted to be surgically removable for minimized susceptibility to MRI artifacts.
An implant magnet arrangement includes multiple cylindrical magnets **300** located within the magnet case **306** and configured to cooperate with a corresponding external holding magnet in an external device located over the overlying skin to magnetically hold the external device against the overlying skin. Each cylindrical implant magnet **300** has a center cylinder axis **301** that is perpendicular to the case rotation axis **307,** and each cylindrical magnet **300** is configured to be rotatable about its center cylinder axis **301.**

Each cylindrical magnet **300** has an outer cylindrical surface **302** with a north magnetic pole and a south magnetic pole. In the most general sense, a "cylindrical surface" is a surface consisting of all the points on all the lines which are parallel to a reference line and which pass through a fixed plane curve in a plane not parallel to the given line. In the present disclosure, the cylinder is a so-called right circular cylinder, in which the "fixed plane curve" is a circle, and the reference line is a line that is perpendicular to circle plane, for example the center cylinder axis 301. A north magnetic direction **303** is defined by a radial vector extending from the center cylinder axis **301** to the north magnetic pole. And a similar south magnetic direction **304** is defined by a radial vector extending from the south magnetic pole to the center cylinder axis **301**. The north magnetic pole and the south magnetic pole are arranged with respect to each other so as not to lie on a common diameter through the center cylinder axis **301** such that the north magnetic direction **303** and the south magnetic direction **304** form a "magnetic angle" $\alpha$ that is less than 180 degrees with a vertex at the center cylinder axis **301**. For example, the magnetic angle $\alpha$ may specifically be between 90° and 140° (or some other defined range). Such magnetic angle $\alpha$ can be established for example by forming the cylindrical magnet **300** from two preformed portions **309** and **310** which are magnetized according to the above-mentioned north and south magnetic directions **303, 304** in a manner that will be explained with reference to a further embodiment in more detail below. In the embodiment shown in Fig. 3, the preformed portions **309, 310** each correspond to a longitudinal half of the full cylindrical magnet **300** which are attached to each other along an interface indicated by the dashed line **311** in Fig. 3A.

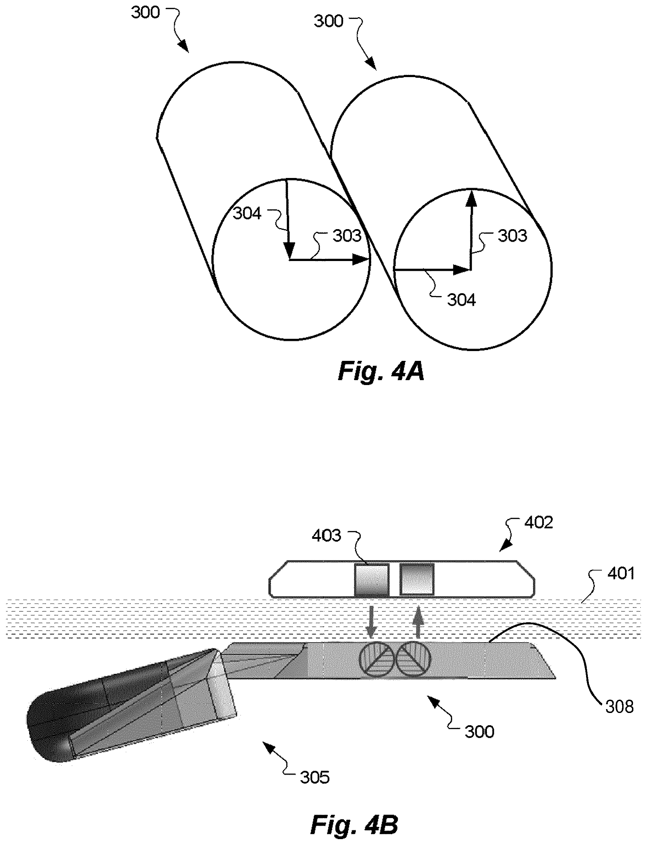

Figures 4A and 4B show how the magnetic fields of the cylindrical magnets **300** align to cooperate with one or more external holding magnets **403** in an external device **402** where the cylindrical magnets **300** magnetically align with respect to each other to create a common line of magnetic flux through the magnets, the magnet case, and the overlying skin to cooperate with the external holding magnet 403. Since the two cylindrical magnets **300** are arranged closely together (e.g., less than 2 mm between their outer cylindrical surfaces **302**), the two adjacent magnetic poles form an attractive magnetic connection with a common magnetic direction that is parallel to the outer surface **308** of the implant device **305** and the overlying skin **401**. Due to the V-shaped magnetic angle, the magnetic direction of the non-adjacent halves of the two cylindrical magnets **300** guides the magnetic flux towards the outer surface **308** of the implant device **305** and the overlying skin **401,** thereby allowing a strong magnetic attraction with the external holding magnet **403** that is almost as strong as with older-type axially magnetized implant magnets that have a magnetization direction that is also normal to the skin **401.**

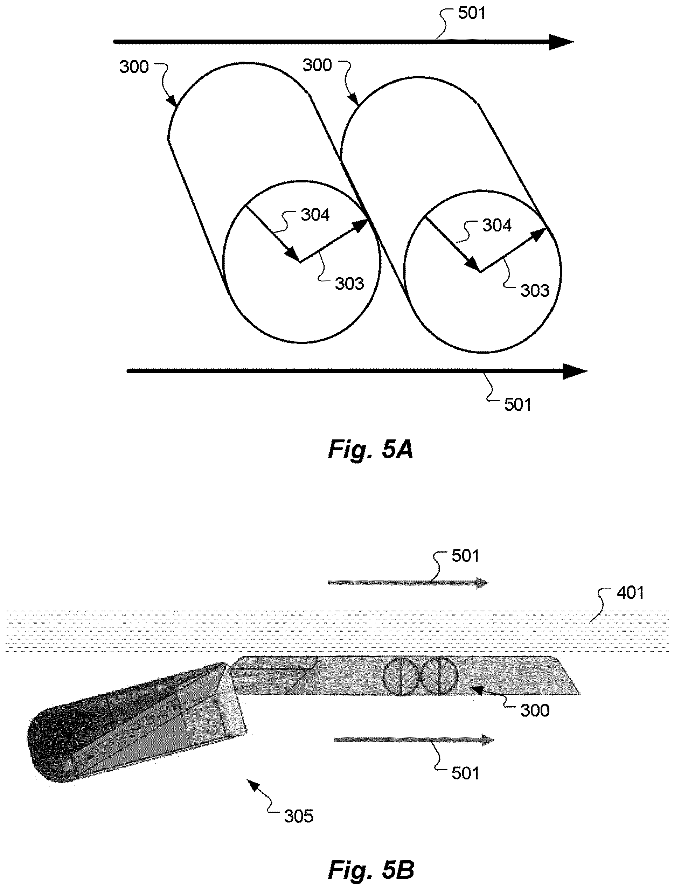

Figures 5A and 5B show how the cylindrical magnets **300** align in the presence of an MRI magnetic field **501**. As can be seen, the two cylindrical magnets **300** immediately align relative to the external magnetic field **501** so that the torque generated by the respective magnetizations of the different halves of each magnet **300** interacting with the external magnetic field **501** cancels out. In Fig. 5B to 7B, the magnetization is schematically represented by the hatched lines.

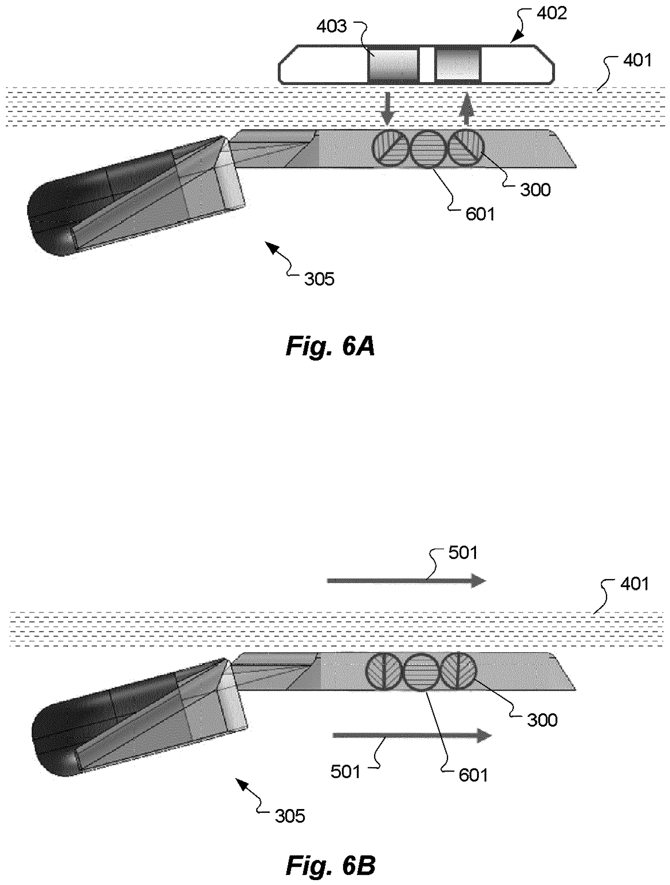

Figures 6A and 6B show an example of an embodiment with a diametrically magnetized supplemental cylindrical magnet **601** that is located between the cylindrical magnets **300**. The supplemental cylindrical magnet **601** is configured to couple the common line of magnetic flux between the cylindrical magnets **300**. This provides an increased distance between the two local maxima of the magnetic flux through the skin **401** thus improving the magnetic attraction to the external magnet **403**.

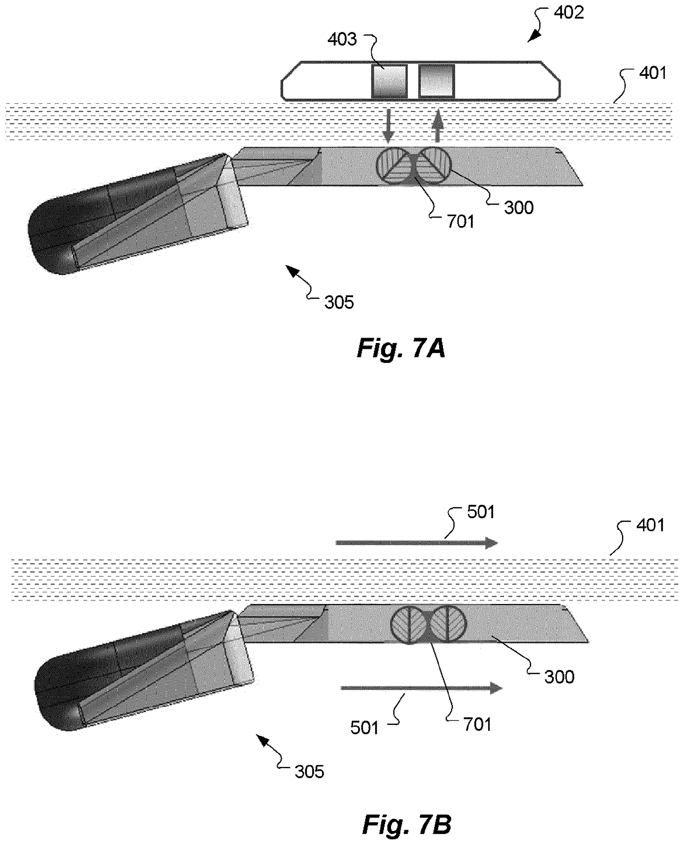

Figures 7A and 7B show an example of an embodiment with soft magnetic material **701** that is located between the cylindrical magnets **300** to couple the common line of magnetic flux between the cylindrical magnets **300**.

In the embodiments described above, the cylindrical magnets **300** are configured to be fully rotatable about the center cylinder axis **301** through a complete rotation range of 360 degrees, and so that the magnet case **306** containing the cylindrical magnets **300** can turn around its case axis **307**. Otherwise, when the center cylinder axis **301** were fixed and a strong external magnetic field **501** is oriented anti-parallel to the cylindrical magnets **300,** the magnets would flip by 180° and the magnetically-strong side of the magnets would then face towards the underlying skull in a medial direction instead of towards the skin in lateral direction. Note that the orientation of the external magnetic field **501** in a given MRI scanner is different when an implant user is scanned with the head first versus with legs first. Accordingly, it is advantageous if the magnet arrangement can handle both of these orientations of the external magnetic field **501**. Moreover, there is no general convention of the orientation of the external magnetic field **501** in MRI scanners, and in some cases where two MRI scanners are arranged next to each other in the same facility, the orientations of the respective external magnetic fields **501** are even deliberately chosen to be of opposite orientation.

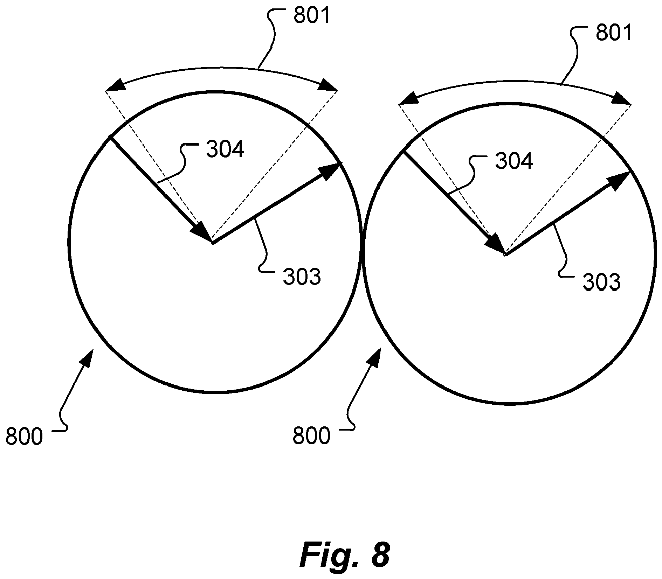

Still, a further design variant with two cylindrical magnets in V-shaped magnetization also works when the magnets have only one degree of freedom and have a rotation angle limited to about 90° only. Figure 8 shows an embodiment of cylindrical implant magnets **800** that are configured to be limitedly rotatable through a limited rotation range **801** of less than 180 degrees according to another embodiment of the present invention. For example, the cylindrical implant magnets may have a V-shaped magnetization with a magnetic angle between 100° and 140°, and the limited rotation range **801** may be 90°.

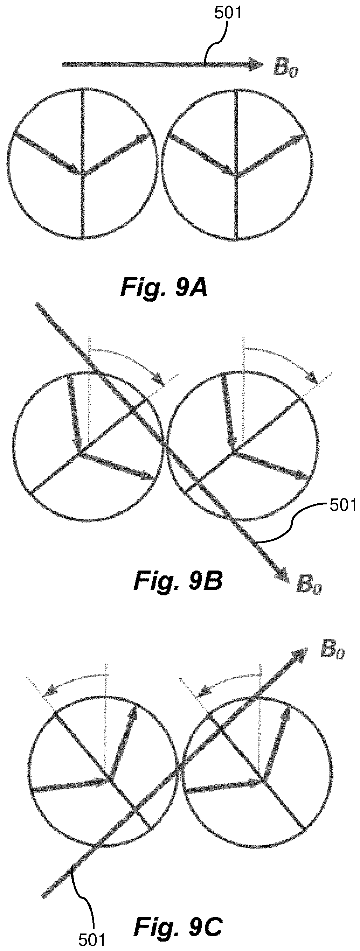

Figures 9A-9C show orientations of the limited rotation range cylindrical implant magnets **800** for different orientations of an external MRI magnetic field **501**. As long as the strong external magnetic field **501** has a component oriented parallel to the overall magnetization of the cylindrical implant magnets **800**, the magnets behave the same as with the other embodiments described above.

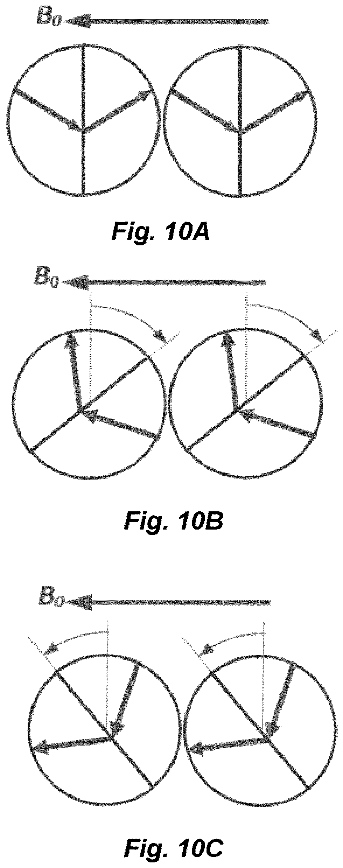

Figures 10A-10C show orientation of the limited range cylindrical implant magnets for reverse magnetization in a reduced rotatability embodiment of the present invention. When a strong external magnetic field is oriented antiparallel to the overall magnetization of the cylindrical implant magnets **800,** they cannot turn by roughly 180° to align to be parallel with the external magnetic field. Instead, each implant magnet **800** reverses its magnet polarity.

In the embodiments described above, despite some individual parts of the implant magnet being oriented perpendicular to the skin surface, the magnets do not weaken in an MRI environment because they immediately turn into a safe orientation relative to the strong static magnetic field of the MRI scanner. Each individual magnet always has a component parallel to the strong static magnetic field of the MRI scanner. And so each individual magnet always aligns such that there is no torque to the outside. The magnetic flux is directed to the skin side and reduced in medial direction. Therefore, the MRI artefact reaches less into the medial direction and is more oriented towards the skin side.

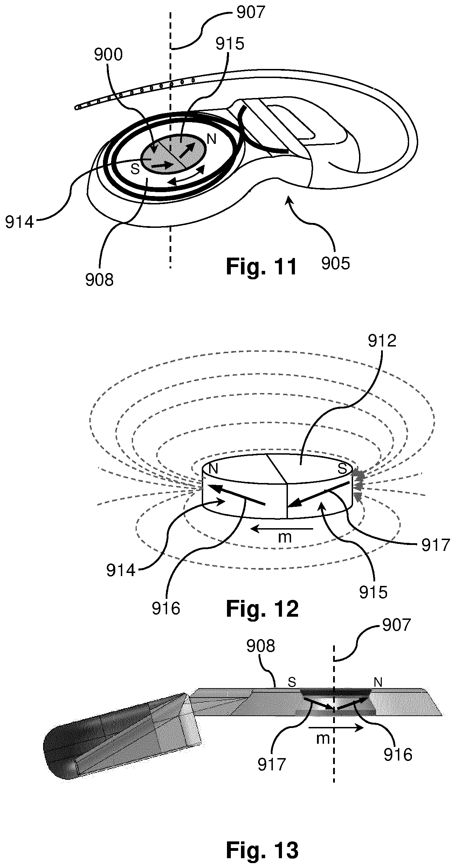

Shown in Fig. 11 is an example of a further implant device, in the specific embodiment a cochlear implant device **905** that is generally similar to the cochlear implant device **305** of Fig. 3B. The cochlear implant device **905** contains signal processing circuitry (not shown) configured for receiving an implant communications signal transmitted from an external device, such as an external device as shown under reference sign **402** in figures 4B and 6A through overlying skin 401 of an implanted patient.

The implant device **905** includes an outermost surface **908** adapted to lie between the overlying skin **401** and underlying skull bone and is at least approximately parallel to the skin **401** of the implanted patient. The implant device **905** further comprises an implant magnet **900** configured to cooperate with an external holding magnet in an external device **402** to be located over the overlying skin **401** to magnetically hold the external device against the overlying skin **401.**

As indicated in Fig. 11 to 13, the implant magnet **900** has a north magnetic pole, a south magnetic pole, and as a whole has an overall magnetic dipole moment $m$ that is parallel to the outermost surface **908** and hence parallel to the skin **401** (not shown in Fig. 11 to 13). The overall magnetic dipole moment $m$ need not be precisely parallel to the outermost surface **908** (the skin **401**), but should be at least approximately parallel to it, for example form an angle of 30° or less, preferably 20° or less with respect to said outermost surface **908.**

The implant magnet **900** is rotatable around a rotation axis **907** that in the embodiment shown is perpendicular to said outermost surface **908** so that in each available rotational position of said implant magnet **900** upon rotation around its rotation axis **907,** the overall magnetic dipole moment $m$ is parallel to said outermost surface **908.**

As is seen in Fig. 12 and 13, the implant magnet **900** has an outer end surface **912** facing said outermost surface **908** of said implant device **905** (i.e. towards the skin **401** in the implanted state), and an inner end surface **913** facing away from said outermost surface **908** (i.e. towards the inside of a patient head in the implanted state). Both of the outer and inner end faces **912, 913** are planar surfaces and are perpendicular to said rotation axis **907.** For example, the implant magnet **900** may have a cylindrical disc shape, in which the side face is formed by a cylindrical surface, as shown in Fig. 12. However, the side face need not be precisely cylindrical, but could have a slightly conical shape, as shown in Fig. 13. It is however preferred that said implant magnet **900** has a shape that is rotationally symmetric around said rotation axis **907.**

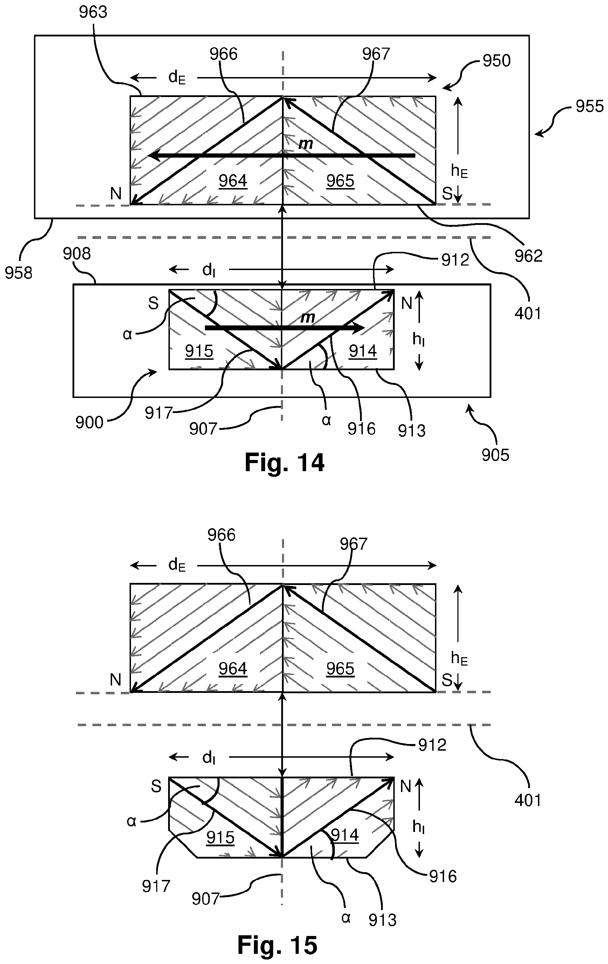

Moreover, the implant magnet **900** has a north end portion **914** including said north magnetic pole and a south end portion **915** including said south magnetic pole. Both of said north and south end portions **914, 915** are formed from permanent magnetic material and each have an individual magnetic dipole moment **916, 917** that is inclined with respect to said overall magnetic dipole moment m, as will be explained in more detail with reference to Fig. 14.

Fig. 14 again schematically shows an implant device **905** in a sectional view including an implant magnet **900,** as well as an external device **955** comprising an external magnet **950.** By magnetic interaction between the internal and external magnets **905, 950,** the external device **955** may be attached to the skin **401** of a patient. The internal magnet **900** is comprised of two halves, one being formed by the north end portion **914** and the other by the south end portion **915.** The hatched lines with arrowheads indicate the local magnetization **M.** It is seen that the local magnetizations in the north and south end portions **914, 915** have deviating directions, thereby leading to what was referred to as the "magnetic angle" with reference to the first aspect of the invention above. Each of the north and south end portions **914, 915** has an individual magnetic dipole moment **916, 917,** which corresponds to the space integral over the magnetization **M** in the respective portion. It is then seen that although the overall dipole moment $m$ of the implant magnet **900** as a whole is directed parallel to the outermost surface **908** of the implant device **905,** each of the individual magnetic dipole moments **916, 917** are inclined with respect to the overall dipole moment $m$.

More precisely, it is seen that the magnetic dipole moment **916** in said north end portion **914** is inclined in a plane perpendicular to the outermost surface **908**/skin **401,** to have a component pointing towards said outermost surface **908,** and said individual magnetic dipole moment in said south end portion **915** is inclined to have a component pointing away from said outermost surface. This leads to a situation where the bigger part of the magnetic flux $B$ generated by said implant magnet **901** is located to the outside, where it is needed for generating a holding force holding the external magnet **950.**

In particular, in preferred embodiments, when simply regarding the internal magnet **900** in isolation, i.e. without presence of the external magnet **950** and when placed in air or vacuum, at least 55 %, preferably at least 65 % or even 70 % or more of the total magnetic flux **B** of a magnetic field generated outside of the implant magnet **900** is located "outside" of a middle plane arranged at equal distances from the outer and inner end surfaces **912, 913.** Herein, "outside of the middle plane" means the side at which said outermost surface **908** is located in the assembled state. This way, the holding force can be significantly increased over and implant magnet of the same size and material which would be homogeneously magnetized parallel to the skin **401.**

In the embodiment of Fig. 14, the implant magnet **900** has a diameter $d_I$ in a direction parallel to the overall magnetic dipole moment and a thickness $h_I$ in a direction perpendicular to said outermost surface. In view of this geometry, in both of said north and south end portions **914, 915,** said individual magnetic dipole moment **916, 917** is inclined with respect to said overall magnetic dipole moment **m** by an angle $\alpha = arctan$ ($h_I$ / ($d_I$ /2)). Herein, the angle $\alpha$ is measured in a plane that is perpendicular to the outermost surface **908.** This choice for the angle $\alpha$ is large enough for providing a significant improvement in attraction force with the external magnet **950** over a prior art implant magnet of same size and material that would be homogeneously magnetized parallel to the skin **401.** A significantly larger angle $\alpha$ has been found to be less favorable, for two reasons. First, for larger angles a, the distance between the north and south poles would decrease, which in turn would lead to an excessive decrease in holding force with increasing distance from the implant magnet **900.** Secondly, the angle $\alpha$ of inclination between each individual magnetic dipole moment **916, 917** in said north and south end portions **914** and **915** with respect to the overall magnetic dipole moment **m** should generally be no more than 60°, and with a certain safety margin, preferably no more than 50°, such as to avoid a situation in which the implant magnet **900** could be inadvertently weakened or demagnetized in a strong external MRI field in case the patient does not hold his or her head straight during the MRI procedure, but for example tilted to one side by e.g. up to 30°. Preferred ranges for the angle $\alpha$ are $arctan$ ($h_I$ / ($d_I$ /2)) $-15° \leq \alpha \leq arctan$ ($h_I$ / ($d_I$ /2)) + 7°, more preferably $arctan$ ($h_I$ / ($d_I$ /2)) $-10° \leq \alpha \leq arctan$ ($h_I$ / ($d_I$ /2)) + 5°, provided that in each case, preferably $\alpha \leq 50°$.

Note that the external magnet **950** in the external device **955** of the embodiment of Fig. 14 has a similar structure than the internal magnet **905.** That is to say, the external magnet **950** has an overall magnetic dipole moment **m** that is parallel to the innermost surface **958** of the external device **955** that lies adjacent to the skin **401.** The external magnet **950** likewise has a north end portion **964** and a south end portion **965** in which the respective individual magnetic dipole moment **966, 967** is inclined with respect to the overall dipole moment **m,** such that the individual magnetic dipole moment **966** in said north end portion **964** has a component pointing towards the innermost surface **958** of the external device **955,** and such that the individual magnetic dipole moment **967** in said south end portion **965** has a component pointing away from said innermost surface **958** of the external device **955.** However, while the implant magnet **905** has been specifically devised for compatibility with an MRI external magnetic field, the external device **955** can be taken off the cochlear implant user prior to the MRI procedure, such that it does not need to employ a similar design. In particular, the external magnet **950** need not be rotatable, and it is also not necessary that its overall magnetic dipole moment **m** is parallel to the innermost so face **958** (skin **401**). Nevertheless, in preferred embodiments, the design of the external magnet **950** is similar to that of the internal magnet **905,** incorporating some or all of the features that were described in the above summary of the invention.

Fig. 15 shows an embodiment which is similar to that of Fig. 14, with the main difference that the edges of the implant magnet **905** at the inner end surface **913** are chamfered. This allows for an improved magnetic flux, and further allows for concentrating more than half of the magnet mass towards the outside of the middle plane, i.e. on a side of said middle plane at which said outermost surface **908** is located.

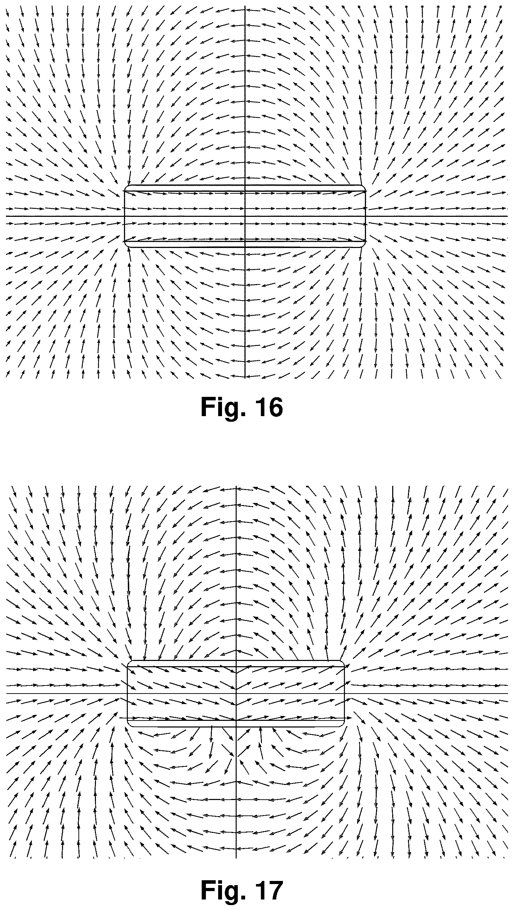

Fig. 16 illustrates the direction of the magnetic flux generated by an ordinary implant magnet that is homogeneously magnetized. In contrast to this, Fig. 17 illustrates the direction of the magnetic flux generated by an implant magnet **900** of the type shown in Fig. 11 to Fig. 14 having north and south end portions **914, 915** with individual magnetic dipole moments **916, 917** that are inclined with respect to the overall magnetic dipole moment of the implant magnet **900.**

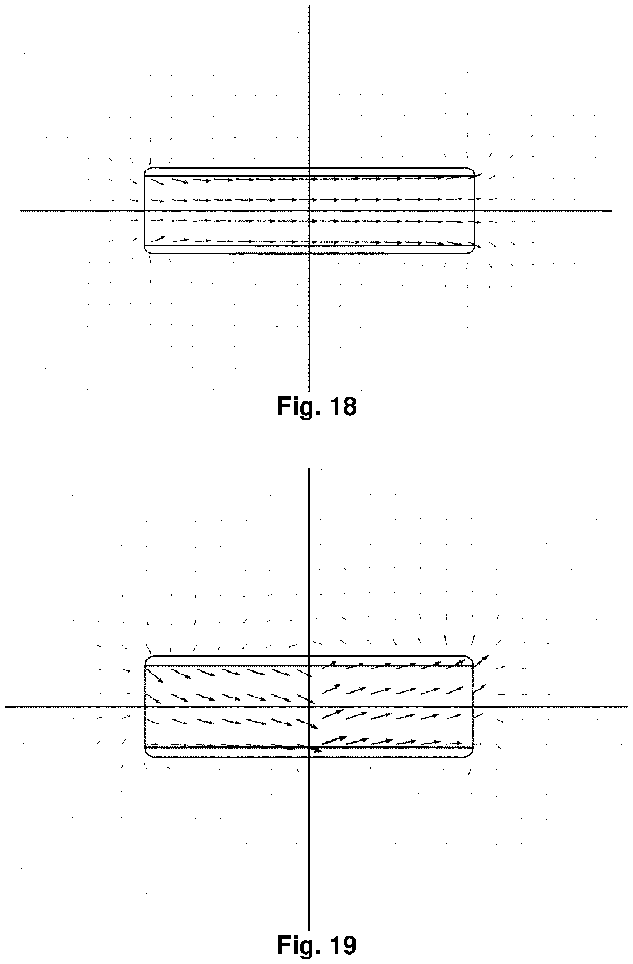

Fig. 18 illustrates the flux density of the ordinary implant magnet of Fig. 16. In other words, while Fig. 16 only shows the direction of the magnetic flux, Fig. 18 indicates the flux density, which is represented by the size of the arrows displayed in the diagram. It is seen that both, the magnetic flux direction and the magnetic flux density are mirror symmetric with respect to the aforementioned middle plane of the magnet, which is a consequence of the homogeneous magnetization. Fig. 19 shows the flux density of the implant magnet **900** of the invention of Fig. 17. It is seen that in the implant magnet **900** of the invention, the poles are shifted "upwards" in the representation of Fig. 19, such that they are located above the middle plane. Indeed, as the skilled person will appreciate, the poles correspond to the regions where the flux density at the surface of the implant magnet **900** is the highest, and these locations are found at the upper left and right corners in the representation of Fig. 19, which is hence consistent with what is schematically shown in Fig. 14 and 15. Moreover, it is seen that of the total magnetic flux of the magnetic field generated outside the implant magnet **900** when taken in isolation, the bigger part is located above the middle plane. Accordingly, the magnetic field generated by the implant magnet **900** of the invention is indeed suitable for generating higher attraction forces when cooperating with an external magnet.

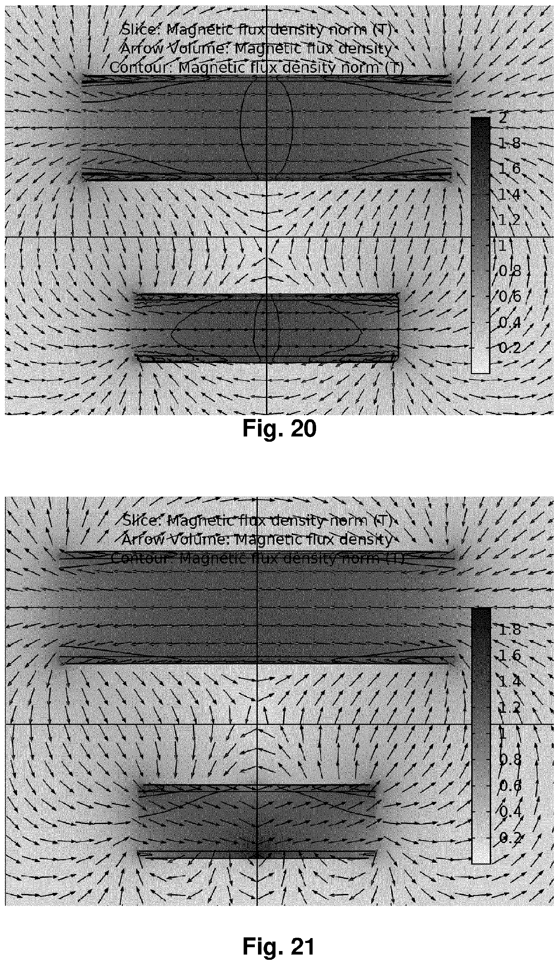

Fig. 20 shows the magnetic flux density generated upon the interaction of a homogeneously magnetized external magnet and the ordinary implant magnet of Fig. 16 and 18. For comparison, Fig. 21 shows the magnetic flux density generated upon the interaction of the same homogeneously magnetized external magnet as in Fig. 20 and the implant magnet **900** of Fig. 17 and 19. It is seen that using the implant magnet **900** of the invention, the attraction force per unit volume of the implant magnet 900 could be increased by 15 %. This gain in attraction force can be even increased further if an external magnet of the type shown under reference sign **950** in Fig. 14 and 15 is used.

In the embodiment shown, both the implant magnet **900** and the external magnet **950** are manufactured from two separate anisotropic magnet pieces which form the north and south end portions **914, 915; 964, 965** in the finished magnet **900, 950.** The anisotropic magnet pieces each have a preferred magnetization direction that corresponds to the direction of the individual magnetic dipole moment **916, 917; 966, 967** in the finished magnet **900, 950.** The preferred magnetization direction can be imprinted on the magnet material by applying a corresponding magnetic field upon its manufacture, for example during a corresponding sintering process. The respective magnet pieces can be joined for example by gluing them together, and only after joining them, the final magnetization is established by applying a strong magnetization pulse parallel to the direction of the overall dipole moment of the finished magnet **900, 950.** Due to the anisotropic character of the magnet pieces, this magnetization pulse will not magnetize both pieces along the direction of the magnetic field of the strong magnetization pulse, but will magnetize them according to their preferred magnetization directions. In preferred embodiments, the implant magnet **900** and/or the external magnet **950** is a rare earth magnet, in particular a rare earth magnet comprising neodymium, samarium, terbium, dysprosium or holmium.

In the embodiments of both, Fig. 14 in Fig. 15, the north and south end portions **914, 915; 964, 965** are directly adjacent with each other, each forming one of two halves of the implant magnet **900** and external magnet **950,** respectively.

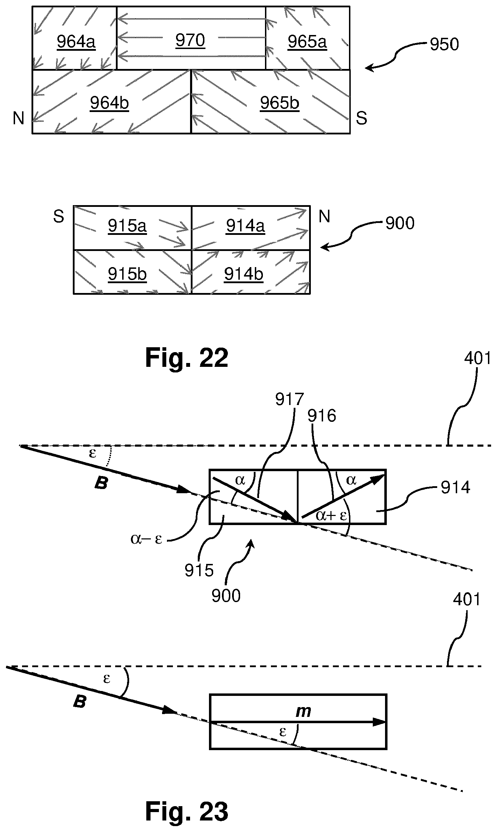

In alternative embodiments, however, north and south end portions of said implant magnet may be separated from each other by an intermediate portion having an individual magnetic dipole moment that is (at least approximately) parallel to said overall magnetic dipole moment. An example for this is shown with respect to the external magnet **950** in Fig. 22, where such intermediate portion is shown at reference sign **970.** This intermediate portion **970** allows for comparatively large inclinations of the magnetization in the north and south end portions **964, 965** while at the same time avoiding magnetic short-circuits at the outer surface, thereby leading to very good holding forces.

In addition or alternatively, one or both of said north and south end portions **914, 915** of said implant magnet **900** may have an outer section **914a, 915a** closer to the outermost surface **908** (not shown in Fig. 22, see Fig. 14) and an inner section **914b, 915b** further away from said outermost surface **908,** and an angle of inclination of the individual magnetic dipole moment (indicated by the magnetization in Fig. 22) with respect to the overall magnetic dipole moment in said outer section **914a, 915a** is less than in said inner section **914b, 915b.** This embodiment allows for an improved magnetic flux within the implant magnet **900** while avoiding the distance of the north and south poles to decrease. A similar design is likewise indicated for the external magnet **950** in Fig. 22.

Fig. 23 shows in the lower half a conventional implant magnet having a homogeneous magnetization and a dipole moment **m** that is parallel to the skin **401** and that is rotatable around an axis perpendicular to the skin **401.** When the implanted person is placed in an external magnetic field **B** of an MRI apparatus, it is generally assumed that the magnetic field **B** is parallel to the skin **401,** and under this assumption, the implant magnet can rotate such as to bring its dipole moment **m** in alignment with the external magnetic field **B,** with no torque acting on the implant magnet anymore. However, this is an idealized assumption, because the implanted magnet might not be perfectly parallel to the skin overlying the magnet, and in practice, the skin overlying the magnet will not be precisely parallel to the external magnetic field **B,** due to the individual anatomy of the patient and the fact that the patient may tilt his or her head sideways. Accordingly, in practice a situation as indicated in the upper part of Fig. 23 arises, in which the magnetic dipole moment **m** of the conventional implant magnet will be inclined with respect to the external magnetic field **B** by an angle $\varepsilon$. In this situation, a torque $\tau$ is applied to the implant magnet having a magnitude $|\tau| = |\boldsymbol{m}| \cdot |\boldsymbol{B}| \cdot \sin(\varepsilon)$.

The upper part of Fig. 23 shows the same situation for an implant magnet **900** according to an embodiment of the invention, having north and south end portions **914, 915** forming halves of the implant magnet **900** and having respective individual magnetic dipole moments **916, 917** which are each inclined with respect to the total magnetic dipole moment of the implant magnet **900** by an angle a. This means that in each of the two halves forming the north and south end portions **914, 915,** there will be a local torque applied even if the external magnetic field **B** is parallel to the overall magnetic dipole moment of the implant magnet **900,** but these two local torques cancel each other. Assuming that the magnitude of the individual dipole moment **916, 917** in each of the north and south end portions **914, 916** is $m_o$, the magnitude of the total torque in case of an inclined external magnetic field **B** as illustrated in the lower part of Fig. 23 can be calculated as follows:

$$|\boldsymbol{\tau}| = m_o \cdot |\boldsymbol{B}| \cdot \sin(\alpha + \varepsilon) - m_o \cdot |\boldsymbol{B}| \cdot \sin(\alpha - \varepsilon) = 2 \cdot m_o \cdot |\boldsymbol{B}| \cdot \sin(\varepsilon) \cdot \cos(\alpha).$$

Comparing this torque to the torque experienced by a conventional homogeneously magnetized implant magnet of same dimensions, one can assume that $2 \cdot m_o \approx |\boldsymbol{m}|$, with $|\boldsymbol{m}|$ being again the magnitude of the magnetic dipole moment of the conventional homogeneously magnetized implant magnet. It is therefore seen that the torque experienced by the implant magnet **900** of the invention in the inclined external magnetic field ***B*** is actually reduced by a factor of cos(a) as compared to the conventional magnet of the same size, making the implant magnet **900** of the invention less sensitive to deviations from the idealized assumption of an external magnetic field being parallel to the skin.

Although various exemplary embodiments of the invention have been disclosed, it should be apparent to those skilled in the art that various changes and modifications can be made which will achieve some of the advantages of the invention without departing from the true scope of the invention.

**Claims**

1. A magnet arrangement for a hearing implant device, the arrangement comprising:

   an implant device (305) containing signal processing circuitry configured for receiving an implant communications signal transmitted from an external transmitting coil through overlying skin (401) of an implanted patient, wherein the implant device (305) includes an outermost surface (308) adapted to lie between the overlying skin (401) and underlying skull bone of the implanted patient;
   a magnet case (306) within the implant device (305), wherein the magnet case (306) is configured to be rotatable about a case rotation axis (307) which is at least approximately perpendicular to said outermost surface (308) of the implant device (305); and
   an implant magnet arrangement within the magnet case (306) configured to cooperate with an external holding magnet (403) in an external device (402) to be located over the overlying skin (401) to magnetically hold the external device (402) against the overlying skin (401);
   wherein the implant magnet arrangement comprises a plurality of cylindrical magnets (300), each with a center cylinder axis (301) perpendicular to the case rotation axis, and each cylindrical magnet (300) configured to be rotatable about the center cylinder axis;
   wherein each cylindrical magnet has an outer cylindrical surface with a north magnetic pole and a south magnetic pole;
   wherein a north magnetic direction (304) is defined by a radial vector extending from the center cylinder axis to the north magnetic pole;
   wherein a south magnetic direction (304) is defined by a radial vector extending from the south magnetic pole to the center cylinder axis (301); and
   wherein the north magnetic pole and the south magnetic pole are arranged with respect to each other so as not to lie on a common diameter through the center cylinder axis (301) such that the north magnetic direction (303) and the south magnetic direction (304) form a magnetic angle less than 180 degrees with a vertex at the center cylinder axis (301).

2. The magnet arrangement according to claim 1, wherein the implant magnet arrangement is configured for the cylindrical magnets (300) to magnetically align with respect to each other to create a common line of magnetic flux through the cylindrical magnets (300), the magnet case (306), and the overlying skin (401) to cooperate with the external holding magnet (403),
   wherein the magnet arrangement preferably further comprises:

   one or more diametrically magnetized supplemental cylindrical magnets (601) between the plurality of cylindrical magnets (300) configured to couple the common line of magnetic flux between the plurality of cylindrical magnets (300), and/or
   wherein the magnet arrangement preferably further comprises soft magnetic material (701) between the plurality of cylindrical magnets (300) configured to couple the common line of magnetic flux between the plurality of cylindrical magnets (300).

3. The magnet arrangement according to one of the preceding claims, wherein the implant magnet arrangement is configured to respond to a strong external magnetic field by rotation of the magnet case (306) about the case rotation axis (307) and rotation of the cylindrical magnets (300) about their respective center cylinder axes (301) so as to minimize net torque imparted to the implant device (305), and/or

   wherein the magnetic angle is between 90 degrees and 140 degrees, and/or
   wherein each cylindrical magnet (300) is configured to be fully rotatable about the center cylinder axis (301) through a complete rotation range of 360 degrees, and/or

wherein each cylindrical magnet (300) is configured to be limitedly rotatable about the center cylinder axis (301) through a limited rotation range of less than 180 degrees,

wherein the limited rotation range is preferably 90 degrees.

4. An implant device (905) containing signal processing circuitry configured for receiving an implant communications signal transmitted through overlying skin (401) of an implanted patient, wherein the implant device (905) includes an outermost surface (908) adapted to lie between the overlying skin (401) and underlying skull bone and at least approximately parallel to the skin (401) of the implanted patient; and

an implant magnet (900) configured to cooperate with an external holding magnet (950) in an external device (955) to be located over the overlying skin (401) to magnetically hold the external device (955) against the overlying skin (401);

wherein said implant magnet (900) has a north magnetic pole, a south magnetic pole, and as a whole has an overall magnetic dipole moment that is parallel to or at an angle of 30° or less with respect to said outermost surface (908),

wherein said implant magnet (900) has a north end portion (914) including said north magnetic pole and a south end portion (915) including said south magnetic pole, said north and south end portions (914, 915) each being formed from permanent magnetic material and each having an individual magnetic dipole moment (916, 917) that is inclined with respect to said overall magnetic dipole moment,

wherein said individual magnetic dipole moment (916) in said north end portion (914) is inclined with respect to said overall magnetic dipole moment such as to have a component pointing towards said outermost surface (908), and said individual magnetic dipole moment (917) in said south end portion (915) is inclined with respect to the overall magnetic dipole moment such as to have a component pointing away from said outermost surface (908).

5. The implant device (905) of claim 4, wherein said implant magnet (900) is rotatable around a rotation axis (907) that is perpendicular to said outermost surface (908), or deviates from perpendicular by less than 30°, wherein in each available rotational position of said implant magnet (900) upon rotation around said rotation axis (907), said overall magnetic dipole moment is parallel to or at an angle of 30° or less with respect to said outermost surface (908).

6. The implant device (905) of claim 5, wherein said implant magnet (900) has a shape that is rotationally symmetric around said rotation axis (907), and/or

wherein said implant magnet (900) has an outer end surface (912) facing said outermost surface (908) of said implant device (905) and an inner end surface (913) facing away from said outermost surface (908), wherein one or both of said inner and outer end surfaces (912, 913) are perpendicular to said rotation axis (907).

7. The implant device (905) of one of claims 4 or 5, wherein said implant magnet (900) has a planar outer end surface (912), and/or

wherein the angle of inclination between each individual magnetic dipole moment (916, 917) in said north and south end portions (914, 915) with respect to the overall magnetic dipole moment is $\leq 50°$, and/or

wherein said implant magnet (900) has an average diameter $d_I$ in a direction parallel to the overall magnetic dipole moment and an average thickness $h_I$ in a direction perpendicular to said outermost surface (908), wherein in one or both of said north and south end portions (914, 915), said individual magnetic dipole moment (916, 917) is inclined with respect to said overall magnetic dipole moment by an angle a, wherein

$$\arctan\left(h_I / (d_I / 2)\right) - 15° \leq \alpha \leq \arctan\left(h_I / (d_I / 2)\right) + 7°,$$

preferably

$$\arctan\left(h_I / (d_I / 2)\right) - 10° \leq \alpha \leq \arctan\left(h_I / (d_I / 2)\right) + 5°.$$

8. The implant device (905) of one of claims 4 to 7, wherein said north and south end portions (914, 915) are directly adjacent with each other, and in particular each form one of two halves of said implant magnet (900), and/or

wherein said north and south end portions (914, 915) of said implant magnet (900) are separated from each other by an intermediate portion having an individual magnetic dipole moment that is parallel to said overall magnetic dipole moment, or deviates from parallel by less than 10°, preferably less than 5°, and/or wherein one or both of said north and south end portions (914, 915) of said implant magnet (900) has an outer section (914a, 915a) closer to said outermost surface (908) and an inner section (914b, 915b) further away from said outermost surface (908), wherein an angle of inclination of the individual magnetic dipole moment with respect to the overall magnetic dipole moment in said outer section (914a, 915a) is less than in said inner section (914b, 915b).

9. The implant device (905) of one of claims 4 to 8, wherein said implant magnet (900) has an outer end surface (912) facing said outermost surface (908) of said implant device (905) and an inner end surface (913) facing away from said outermost surface (908), wherein a middle plane is defined to be located at equal distance from said outer and inner end surfaces (912, 913), and wherein said implant magnet (900) fulfils one or both of the following criteria (i) and (ii):

(i) at least 55 %, preferably at least 65 % of the total magnetic flux of a magnetic field generated outside of the implant magnet when placed in isolation in air or a vacuum is located on a side of said middle plane at which said outermost surface (90) is located in the assembled state,
(ii) more than 50%, preferably more than 55% of the mass of the magnet (900) is located on a side of said middle plane at which said outermost surface (908) is located, wherein in particular, the edges of the magnet (900) at the inner end surface (913) are chamfered, and/or

wherein said north and south end portions (914, 915) are formed from anisotropic magnet elements each having a preferred magnetization direction, wherein said anisotropic magnet elements are joined with each other or with an intermediate portion arranged in between, wherein said preferred magnetization directions are arranged at an angle with respect to the overall dipole moment of the implant magnet as a whole.

10. The implant device (905) of one of claims 4 to 9, wherein on at least part of the inner end surface (913) a layer of magnetic soft material, such as for example soft iron, is applied, and/or wherein said implant magnet (900) is a rare earth magnet, in particular a rare earth magnet comprising neodymium, samarium, terbium, dysprosium or holmium.

11. An implant system comprising an implant device (905) or magnet arrangement according to one of the preceding claims and an external device (955), said external device (955) comprising signal processing circuitry configured for transmitting an implant communications signal to said implant device (905), said external device (955) comprising an innermost surface (958) adapted to lie adjacent to said skin (401); and an external magnet (950) or magnet assembly in said external device (955) to be located over the overlying skin (401) and magnetically configured to cooperate with the implant magnet (900) of said implant device (905) or with said magnet arrangement such as to hold the external device (955) against the skin (401).

12. The implant system of claim 11, wherein said external magnet (950) or magnet arrangement has a north magnetic pole, a south magnetic pole, and as a whole has an overall magnetic dipole moment that is parallel to or at an angle of 30° or less with respect to said innermost surface (958) of said external device (955).

13. The implant system of claim 12, wherein said external magnet (950) has a north end portion (964) including said north magnetic pole and a south end portion (965) including said south magnetic pole, said north and south end portions (964, 964) each being formed from permanent magnetic material and each having an individual magnetic dipole moment that is inclined with respect to said overall magnetic dipole moment of said external magnet (950), wherein said individual magnetic dipole moment (966) in said north end portion (964) has a component pointing towards said innermost surface (958) of said external device (955), and said individual magnetic dipole moment (967) in said south end portion (965) has a component pointing away from said innermost surface (958) of said external device (955).

14. The implant system of one of claims 11 to 13, wherein said external magnet (950) is rotatable around a rotation axis that is perpendicular to said innermost surface (958) of said external device (955) or deviates from perpendicular by less than 30°, wherein in each available rotational position of said external magnet (950) upon rotation around said rotation axis, said overall magnetic dipole moment is parallel to or at an angle of 30° or less with respect to said innermost surface (958), wherein said external magnet (950) preferably has a shape that is rotationally symmetric around its rotation axis, and/or

wherein said north and south end portions (964, 965) of said external magnet (950) are directly adjacent with each other, and in particular each form one of two halves of said external magnet (950), and/or
wherein said external magnet (950) is a rare earth magnet, in particular a rare earth magnet comprising neodymium, samarium, terbium, dysprosium or holmium, wherein said external magnet (950) has a planar inner end surface (962) facing said innermost surface (958) of said external device (955).

15. The implant system of one of claims 13 or 14, wherein said external magnet (950) has an average diameter $d_E$ in a direction parallel to the overall magnetic dipole moment and an average thickness $h_E$ in a direction perpendicular to said innermost surface (958) of the external device (955), wherein in one or both of said north and south end portions (964, 965), said individual magnetic dipole moment (966, 967) is inclined with respect to said overall magnetic dipole moment by an angle a, wherein

$$arctan\ (h_E\ /\ (d_E\ /2))\ \text{-}15° \le α \le arctan\ (h_E\ /\ (d_E\ /2))\ + 7°,$$

preferably

$$arctan\ (h_E\ /\ (d_I\ /2))\ \text{-}10° \le α \le arctan\ (h_E\ /\ (d_E\ /2))\ + 5°,$$

and/or

wherein said north and south end portions (964, 965) of said external magnet (950) are separated from each other by an intermediate portion (970) having an individual magnetic dipole moment that is parallel to said overall magnetic dipole moment of said external magnet or deviates from parallel by less than 10°, preferably less than 5°, and/or
wherein one or both of said north and south end portions (964, 966) of said implant magnet (950) have an inner section (964a, 965a) closer to said innermost surface (958) of said external device (955) and an outer section (964b, 965b) further away from said innermost surface (958) of said external device (955), wherein an angle of inclination of the individual magnetic dipole moment with respect to the overall magnetic dipole moment in said inner section (964a, 965a) is less than in said outer section (964b, 965b), and/or
wherein said external magnet (950) has an inner end surface (962) facing said innermost surface (958) of said external device (955) and an outer end surface (963) facing away from said innermost surface (958) of said external device (955),
wherein a middle plane is defined to be located at equal distance from said outer and inner end surfaces (962, 962) of said external device (955), and wherein said external magnet (950) fulfils one or both of the following criteria (i) and (ii):

(i) at least 55 %, preferably at least 65 % of the total magnetic flux of a magnetic field generated outside of the external magnet (950) when placed in isolation in air or a vacuum is located on a side of said middle plane at which said innermost surface of the external device is located in the assembled state,
(ii) more than 50%, preferably more than 55% of the mass of the external magnet (950) is located on a side of said middle plane at which said innermost surface (958) is located, wherein in particular, the edges of the magnet at the inner outer surface are chamfered, and/or

wherein said north and south end portions (964, 965) of said external magnet (950) are formed from anisotropic magnet elements each having a preferred magnetization direction, wherein said anisotropic magnet elements are joined with each other or with an intermediate portion arranged in between, wherein said preferred magnetization directions are arranged at an angle with respect to the overall dipole moment of the external magnet as a whole.

**Fig. 1**

**Fig. 2**

**Fig. 3A**

**Fig. 3B**

**Fig. 4A**

**Fig. 4B**

**Fig. 5A**

**Fig. 5B**

**Fig. 6A**

**Fig. 6B**

*Fig. 7A*

*Fig. 7B*

**Fig. 8**

Fig. 9A

Fig. 9B

Fig. 9C

$B_0$

**Fig. 10A**

$B_0$

**Fig. 10B**

$B_0$

**Fig. 10C**

**Fig. 11**

**Fig. 12**

**Fig. 13**

**Fig. 14**

**Fig. 15**

Fig. 16

Fig. 17

Fig. 18

Fig. 19

Fig. 20

Fig. 21

**Fig. 22**

**Fig. 23**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7566296 B
- US 20110022120 A
- US 8634909 B